# EUROPEAN PATENT SPECIFICATION

(11) **EP 1 161 272 B1**
(45) Date of publication and mention of the grant of the patent: **04.10.2006**
(21) Application number: 00909515.9
(22) Date of filing: 14.03.2000
(51) Int. Cl.: A61M 5/30

(54) **NEEDLELESS SYRINGE**
NADELLOSE SPRITZE
SERINGUE SANS AIGUILLE

(30) Priority: 15.03.1999 GB 9905933
(43) Date of publication of application: 12.12.2001
(73) Proprietor: PowderJect Research Limited, Oxford OX4 4GA (GB)
(72) Inventor: BELLHOUSE, Brian John, Islip, Oxfordshire OX5 2SQ (GB); MILLWARD, Huw Richard, Greater Leys, Oxford OX4 5GE (GB); NABULSI, Samih Muhib, Bishops Stortford, Herts CM22 7QT (GB); PHILLIPS, Monisha Jane, Headington, Oxford OX3 0AW (GB)
(74) Representative: Price, Nigel John King
(86) International application number: PCT/GB2000/000932
(87) International publication number: WO 2000/054827

(56) References cited:
- WO-A-96/20022
- WO-A-96/25190
- WO-A-99/04838
- US-A- 3 595 231
- US-A- 5 026 343
- US-A- 5 204 253
- US-A- 5 630 796

## Description

### Technical Field

The present invention relates generally to a needleless syringe for use in delivering particles into a target surface. More particularly, the invention is drawn to a needleless syringe system configured for delivery of particles initially disposed upon a first surface of a diaphragm using a shockwave force that is imparted upon a second, opposing surface of the diaphragm.

### Background of the Invention

In commonly owned U.S. Patent No. 5,630,796, a particle delivery system is described that entails the use of a needleless syringe. The syringe is used for delivering particles (powdered compounds and compositions) to skin, muscle, blood or lymph. The syringe can also be used in conjunction with surgery to deliver particles to organ surfaces, solid tumors and/or to surgical cavities (e.g., tumor beds or cavities after tumor resection).

The needleless syringe of U.S. Patent No. 5,630,796 is typically constructed as an elongate tubular nozzle, having a rupturable membrane initially closing the passage through the nozzle adjacent to the upstream end of the nozzle. Particles, usually comprising a powdered therapeutic agent, are located adjacent to the membrane. The particles are delivered using an energizing means which applies a gaseous pressure to the upstream side of the membrane that is sufficient to burst the membrane, thereby producing a high velocity gas flow through the nozzle in which the particles are entrained.

Another particle delivery system that entails the use of a needleless syringe is described in commonly owned International Publication Nos. WO 96/20022 and WO 96/25190. The needleless syringes of International Publication Nos. WO 96/20022 and WO 96/25190 generally include the same elements as described above, except that instead of having the particles entrained within a high velocity gas flow, the downstream end of the nozzle is provided with a bistable diaphragm which is moveable between a resting "inverted" position (in which the diaphragm presents a concavity on the downstream face to initially contain the particles) and an active "everted" position (in which the diaphragm is outwardly convex on the downstream face as a result of a shockwave having been applied to the upstream face of the diaphragm). In this manner, the particles initially contained within the concavity of the diaphragm are expelled from the diaphragm at a high initial velocity suitable for delivering the particles into a target surface.

Particle delivery using either of the above-described needleless syringe configurations is carried out with particles having an approximate size that generally ranges between 0.1 and 250 µm. For drug delivery, an optimal particle size is usually at least about 10 to 15 µm (the size of a typical cell). For gene delivery, an optimal particle size is generally substantially smaller than 10 µm. Particles larger than about 250 µm can also be delivered from the device, with the upper limitation being the point at which the size of the particles would cause untoward damage to the target tissue. The actual distance which the delivered particles will penetrate depends upon particle size (e.g., the nominal particle diameter assuming a roughly spherical particle geometry), particle density, the initial velocity at which the particle impacts the target surface, and the density and kinematic viscosity of the target tissue (e.g., skin). In this regard, optimal particle densities for use in needleless injection generally range between about 0.1 and 25 g/cm³, preferably between about 0.8 and 1.5 g/cm³, and injection velocities generally range between about 100 and 3,000 m/sec.

WO 99/04838 discloses the pre-characterizing portion of claim 1.

### Summary of the Invention

Only the embodiments showing and disclosing that the diaphragms have an overall configuration in the shape of a top hat are covered by the present claims.

In one embodiment of the invention, a needleless syringe is provided. The needleless syringe is capable of accelerating particles into a target tissue of a vertebrate subject. The syringe comprises, in operative combination, a body having a lumen extending therethrough. The lumen has an upstream terminus and a downstream terminus, and the upstream terminus of the lumen is interfaced with an energizing means such as a volume of a pressurized driving gas. The syringe further includes a diaphragm arranged adjacent to the downstream terminus of the lumen, wherein the diaphragm has an internal surface facing the lumen and an external surface facing outwardly from the syringe. The diaphragm is moveable between an initial position in which a concavity is provided on the external surface of the diaphragm, and a dynamic position in which the external surface of the diaphragm is substantially convex. The diaphragm is characterized in that its external surface comprises one or more topographical features which selectively retain particles on the external surface of the diaphragm when in its initial, "loaded" position and in that it has an overall configuration in the shape of a top hat.

In another embodiment of the invention, a needleless syringe is provided which comprises a plurality of diaphragms. The diaphragm has a concavity that sealably contains particles comprising a therapeutic agent. In a further embodiment of the invention, a needleless syringe is provided which provides a high pressure shock wave.

These and other embodiments of the present invention will readily occur to those of ordinary skill in the art in view of the disclosure herein.

### Brief Description of the Figures

Some examples of syringes constructed in accordance with the present invention are illustrated in the accompanying drawings, in which:
Figure 1 is an axial section through a first embodiment of the invention;
Figure 2 is a sectional perspective view of the lower part of the syringe of Figure 1 with the addition of a safety plug;
Figures 3-11 are diagrammatic representations of different diaphragm embodiments;
Figure 12 is an axial section through the lower part of a syringe such as that of Figure 1, and shows a catching grid;
Figure 13 is an elevation as seen in the axial direction of the parts shown in Figure 12;
Figures 14-16 show diagrammatically various diaphragm configurations and arrays; and
Figure 17 is an axial section through a needleless syringe.

### Detailed Description of the Preferred Embodiments

Before describing the present invention in detail, it is to be understood that this invention is not limited to particular pharmaceutical formulations or process parameters as such may, of course, vary. It is also to be understood that the terminology used herein is for the purpose of describing particular embodiments of the invention only, and is not intended to be limiting.

All publications, patents and patent applications cited herein, whether *supra* or *infra,* are hereby incorporated by reference in their entirety.

It must be noted that, as used in this specification and the appended claims, the singular forms "a", "an" and "the" include plural referents unless the content clearly dictates otherwise. Thus, for example, reference to "a therapeutic agent" includes a mixture of two or more such agents, reference to "a gas" includes mixtures of two or more gases, and the like.

### A. Definitions

Unless defined otherwise, all technical and scientific terms used herein have the same meaning as commonly understood by one of ordinary skill in the art to which the invention pertains.

The following terms are intended to be defined as indicated below.

The terms "needleless syringe," and "needleless syringe device," as used herein, expressly refer to a particle delivery system that can be used to deliver particles into and/or across tissue, wherein the particles have an average size ranging from about 0.1 to 250 µm, preferably about 10-70 µm. Particles larger than about 250 µm can also be delivered from these devices, with the upper limitation being the point at which the size of the particles would cause untoward pain and/or damage to the target tissue. The particles are delivered at high velocity, for example at velocities of at least about 150 m/s or more, and more typically at velocities of about 250-300 m/s or greater. Such needleless syringe devices were first described in commonly-owned U.S. Patent No. 5,630,796 to Bellhouse et al., incorporated herein by reference, and have since been described in commonly owned International Publication Nos. WO 96/04947, WO 96/12513, and WO 96/20022, all of which publications are also incorporated herein by reference. These devices can be used in the transdermal delivery of a therapeutic agent into target skin or mucosal tissue, either *in vitro* or *in vivo* (*in situ*); or the devices can be used in the transdermal delivery of generally inert particles for the purpose of non- or minimally invasive sampling of an analyte from a biological system. Since the term only relates to devices which are suitable for delivery of particulate materials, devices such as liquid-jet injectors are expressly excluded from the definition of a "needleless syringe."

The term "particles", as used herein, covers a single particle as well as plural particles.

The term "transdermal" delivery captures intradermal, transdermal (or "percutaneous") and transmucosal administration, i.e., delivery by passage of a therapeutic agent into and/or through skin or mucosal tissue. See, e.g., *Transdermal Drug Delivery: Developmental Issues and Research Initiatives,* Hadgraft and Guy (eds.), Marcel Dekker, Inc., (1989); *Controlled Drug Delivery: Fundamentals and Applications,* Robinson and Lee (eds.), Marcel Dekker Inc., (1987); and *Transdermal Delivery of Drugs,* Vols. 1-3, Kydonieus and Berner (eds.), CRC Press, (1987). Aspects of the invention which are described herein in the context of "transdermal" delivery, unless otherwise specified, are meant to apply to intradermal, transdermal and transmucosal delivery. That is, the devices, systems, and methods of the invention, unless explicitly stated otherwise, should be presumed to be equally applicable to intradermal, transdermal and transmucosal modes of delivery.

As used herein, the terms "therapeutic agent" and/or "particles of a therapeutic agent" intend any compound or composition of matter which, when administered to an organism (human or animal) induces a desired pharmacologic, immunogenic, and/or physiologic effect by local and/or systemic action. The term therefore encompasses those compounds or chemicals traditionally regarded as drugs, vaccines, and biopharmaceuticals including molecules such as proteins, peptides, hormones, biological response modifiers, nucleic acids, gene constructs and the like. More particularly, the term "therapeutic agent" includes compounds or compositions for use in all of the major therapeutic areas including, but not limited to, adjuvants, anti-infectives such as antibiotics and antiviral agents; analgesics and analgesic combinations; local and general anesthetics; anorexics; antiarthritics; antiasthmatic agents; anticonvulsants; antidepressants; antigens, antihistamines; anti-inflammatory agents; antinauseants; antineoplastics; antipruritics; antipsychotics; antipyretics; antispasmodics; cardiovascular preparations (including calcium channel blockers, beta-blockers, beta-agonists and antiarrythmics); antihypertensives; diuretics; vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins peptides and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like).

Particles of a therapeutic agent, alone or in combination with other drugs or agents, are typically prepared as pharmaceutical compositions which can contain one or more added materials such as carriers, vehicles, and/or excipients. "Carriers," "vehicles" and "excipients" generally refer to substantially inert materials which are nontoxic and do not interact with other components of the composition in a deleterious manner. These materials can be used to increase the amount of solids in particulate pharmaceutical compositions. Examples of suitable carriers include water, silicone, gelatin, waxes, and like materials. Examples of normally employed "excipients," include pharmaceutical grades of dextrose, sucrose, lactose, trehalose, mannitol, sorbitol, inositol, dextran, starch, cellulose, sodium or calcium phosphates, calcium sulfate, citric acid, tartaric acid, glycine, high molecular weight polyethylene glycols (PEG), and combinations thereof. In addition, it may be desirable to include a charged lipid and/or detergent in the pharmaceutical compositions. Such materials can be used as stabilizers, anti-oxidants, or used to reduce the possibility of local irritation at the site of administration. Suitable charged lipids include, without limitation, phosphatidylcholines (lecithin), and the like. Detergents will typically be a nonionic, anionic, cationic or amphoteric surfactant. Examples of suitable surfactants include, for example, Tergitol® and Triton® surfactants (Union Carbide Chemicals and Plastics, Danbury, CT), polyoxyethylenesorbitans, e.g., TWEEN® surfactants (Atlas Chemical Industries, Wilmington, DE), polyoxyethylene ethers, e.g., Brij, pharmaceutically acceptable fatty acid esters, e.g., lauryl sulfate and salts thereof (SDS), and like materials.

The term "analyte" is used herein in its broadest sense to denote any specific substance or component that one desires to detect and/or measure in a physical, chemical, biochemical, electrochemical, photochemical, spectrophotometric, polarimetric, colorimetric, or radiometric analysis. A detectable signal can be obtained, either directly or indirectly, from such a material. In some applications, the analyte is a physiological analyte of interest (e.g., a physiologically active material), for example glucose, or a chemical that has a physiological action, for example a drug or pharmacological agent.

As used herein, the term "sampling" means extraction of a substance, typically an analyte, from any biological system across a membrane, generally across skin or tissue.

### B. General Methods

In one embodiment of the invention, a needleless syringe device is provided having a body containing a lumen. An upstream end of the lumen is, or is arranged to be, connected to a source of gaseous pressure which can suddenly be released into the lumen. The downstream end of the lumen terminates behind an eversible diaphragm which is movable between an initial, inverted position which provides a concavity for containing particles to be delivered from the device, and an everted, outwardly convex, position. The eversible diaphragm is arranged such that, when an energizing gas flow is released into the lumen, the diaphragm will travel from its inverted to its everted position, thereby projecting the particles from the diaphragm toward a target surface.

The body can be made from any suitably resilient material, preferably from a medical-grade plastic which may be injection-molded into any desired configuration. In addition, any number of suitable energizing means can be used to power the device. For example, a chamber containing a reservoir of compressed gas can be arranged at (interfaced with) the upstream end of the lumen. The gas can be released from the energizing chamber by way of a valve, such as a spring-loaded ball valve or a piston valve, which valves are typically actuated by either mechanical means or by manual manipulation, for example, by movement of two parts of the syringe relative to each other. Alternatively, an energizing chamber can be adapted to provide for a controlled build-up of gaseous pressure from an upstream or associated (local or remote) source. For example, release of a pressurized gas flow may be achieved by building up pressure behind a rupturable membrane until the pressure difference across the membrane is sufficient to rupture the membrane and release the gas suddenly into the lumen. The velocity of the shockwave provided by these and other suitable energizing means can be increased if the driving gas is lighter than air, e.g., helium.

It is preferable, however, that the needleless syringe device is powered using a gas cylinder containing a source of compressed gas. Such gas cylinders are typically deep-drawn from aluminum or some other suitable metal or metal alloy, and find use in powering a range of other devices and appliances such as air pistols or beverage dispensers. Needleless syringe devices that are fitted with a gas cylinder are easily operated by creating a breach in a portion of the cylinder such that the compressed gas can rapidly escape therefrom. This breach can be created by the action of an actuation ram or pin which is used to snap off a frangible tip of the gas source. A number of alternative actuation mechanisms can, of course, be used to create the breach in the gas source. For example, a sharp pin or needle can be used to pierce a hole in the gas source or rupture a membrane or other relatively weak portion of the gas source. Alternatively, a trigger mechanism can be used to open a valve which closes off the gas source. These and other suitable actuation schemes and mechanisms will readily occur to the ordinarily skilled artisan upon reading the instant specification.

The needleless syringe of the present embodiment represents a significant improvement over previous such devices in that the diaphragm is adapted to selectively retain and immobilize particles on the external surface thereof while the diaphragm is in its initial, inverted position (i.e., prior to use of the device), while also readily releasing the particles from the surface of the diaphragm when it is moving into its dynamic position (i.e., as the device is fired).

In accordance with the present invention, then, a needleless syringe is provided having a diaphragm that provides for enhanced particle retention, wherein the external surface of the diaphragm comprises one or more topographical features that selectively retain particles on the external surface of the diaphragm when it is in an initial position. The term "topographical" is used herein in its most broad and general sense to denote the presence of any form or feature distinguishable from the surrounding substantially planar or smooth external surface of the diaphragm. Such forms or figures can be raised (e.g., in relief) or recessed with respect to the surrounding external surface of the diaphragm. One advantage of providing the external surface of the diaphragm with such topographical features is that it increases the surface area on which the particles are retained so that more, if not all, of the particles are immobilized in direct contact with the diaphragm, thereby increasing the loading capacity of the diaphragm.

The topographical features may take a wide variety of forms. For example, the features can form or otherwise comprise one or more pockets for retaining the particles. Such pockets may be formed by providing one or more grooves, channels, troughs, hollows, cavities, folds, kinks, or any conceivable combination of these or like features in the external surface of the diaphragm. Alternatively, a series of superficial cuts or scores can be made in the external surface of the diaphragm to provide the pocket(s). The features may be randomly placed about the surface of the diaphragm, or may be placed in an ordered array, for example as a series of concentric annular grooves.

An advantage of retaining the particles in one or more pockets is that when the diaphragm is in its initial position, the external surface of the diaphragm on which the particles are retained will be relatively in compression, as compared to when the diaphragm is in its dynamic position, in which the same downstream surface of the diaphragm will be comparatively in tension. In other words, during the snap eversion of the diaphragm the side walls of the pockets will tend to move apart, reducing the effective retention of the particles so that they are more freely able to be catapulted outwards toward the target surface. The effective "opening" and "closing" of the pockets as the diaphragm moves between its initial and dynamic positions may be utilized in loading the diaphragm with the particles. That is, the diaphragm can initially be manipulated into an everted "fired" position, so that the pockets are "open for reception of the particles. The diaphragm can then be gently returned to an initial "loaded" or "pre-fired" position, effectively "closing" the pockets so as to grip and selectively retain the particles. Alternatively, the diaphragm may be loaded with the particles while in its "pre-fired" position.

The topographical features may alternatively comprise one or more structures that depend from the surface of the diaphragm and extend in an outward direction from the surface. For example, one or more bristles, fingers, protrusions, ribs, fins, structural partition, rings, or any combination of these or like features can be provided on the external surface of the diaphragm. Here again, the features may be randomly placed on the surface of the diaphragm, or provided in an ordered array, for example arranged as a mesh, honey-comb or lattice work. These features can be injection molded into the diaphragm it self, or created by flock-spraying fibers onto the surface of the diaphragm. A further possibility is to provide a fabric or mesh on the downstream surface of the diaphragm. Particular arrays include a single, or a concentric series of annular ribs, fins or protrusions depending and extending from the external surface of the diaphragm.

In a particularly preferred embodiment, the topographical features comprise a series of projecting bristles. These bristles are particularly efficient in that they can be arranged to converge towards one another when the diaphragm is in its initial "loaded" position to selectively grip and retain the particles between them, and to diverge in the dynamic "fired" position of the diaphragm so as to efficiently release the particles. The bristles are also found to be efficient in distributing more evenly over the surface of the diaphragm a slurry containing gold or other heavy microparticles coated with therapeutic agent of interest, for example a peptide or some genetic material like a DNA.

In order to provide for an even, well spread particle distribution at the target tissue surface, the above diaphragms are provided with a central, substantially flat or planar region. Such diaphragms take on a cross-section that is in the shape of a top hat, wherein the annular flange of the section (e.g., the "brim" of the top hat) can be used to locate the diaphragm in position in the syringe. The substantially flat or planar portion assists in providing a parallel or divergent stream of particles which will impinge evenly over an adequate target surface area. It has been found that the effective target surface area over which the particles are spread will be roughly equal to or slightly greater than the area of the flat or planar region of the diaphragm.

The diaphragm can be comprised of any suitably resilient, flexible polymeric material which can withstand the impact of a shock wave traveling in the driven gas at supersonic speed. Exemplary classes of materials include, for example, polyurethane or silicone rubbers. Selection of suitable flexible dome-shaped diaphragms is within the capabilities of the reasonably skilled artisan upon reading this specification, wherein the flexibility of a particular dome can be characterized by the static buckling stress provided by a particular material of a given thickness. In practice, the diaphragm will normally be molded from a plastics material, such as styrene, polyurethane or a polyester-based thermoplastic elastomer such as those sold by Bayer under the tradename of DESMOPAN^{™} (a polyester urethane) and by DuPont under the tradename of HYTREL^{™}. Although the diaphragm may be molded by any appropriate technique, such as compression-molding or thermoforming, precision profiling which may be needed to produce fine topographical details on the external surface of the diaphragm is best carried out using injection-molding techniques.

When the topographical features are provided by a fabric on the external surface of the diaphragm, the diaphragm may be formed by coating a fabric, such as a tightly woven nylon or polyester fabric (e.g., a fabric having warp and weft threads at a frequency of about 34 per cm) on one side with a polymer (e.g., with a polyurethane), and then molding the resultant coated fabric into a desired diaphragm configuration by thermoforming. The fabric thus provides both pockets for selectively retaining the particles, and a degree of reinforcement to the diaphragm.

If desired, the needleless syringe can be provided with a plug inserted into the concavity of the diaphragm when in its initial position in order to further ensure that the particles are not displaced from the diaphragm prior to use. The plug is then removed before firing the device. Also, in order to increase the ability of the diaphragm to securely retain the particles prior to firing, the external surface of the diaphragm may be provided with a tacky, i.e., lightly adhesive, surface or surface treatment. In this regard, a number of polymers suitable for constructing the instant diaphragms from already have natural surface tackiness, for example, Desmopan 385 and ethylene vinyl acetate (EVA). This tackiness can be enhanced by applying an adhesive coating such as trehalose or a silicone oil (e.g., a silicone dispersion manufactured by NuSil) to the external surface of the diaphragm.

Additionally, the needleless syringe device can be provided with structural reinforcements or other features which can help prevent unwanted burst of the diaphragm while under the strain of the shockwave or built-up pressure within the device. For example, a vent hole (e.g., of ≤ 0.5 mm diameter) can be provided in the rupture chamber or shock tube to avoid excessive build-up of gas pressure behind the diaphragm. In this regard, the transient pressure upstream of the diaphragm needs to be sufficient to evert the diaphragm during particle delivery, but can be vented in this manner so as to maintain the pressure below a critical level at which the diaphragm bursts. Alternatively, one can use insert-molding or co-molding techniques to incorporate a reinforcing metal or polymeric plate or grid at the weakest points of the diaphragm in order to minimize the likelihood of diaphragm failure during device operation. A further alternative is to provide an elastic fibre reinforcement within the diaphragm material. The addition of elastic or Lycra-like fibers within the matrix of a base polymer helps to strengthen the resulting elastomer. When used for the diaphragm, such thermoplastic elastomers would not prevent the diaphragm from expanding past its final, relaxed everted position under the action of the high pressure gas but would allow the diaphragm to relax back to its "fired" position as the gas is vented. In some cases, the diaphragm could relax back to its original inverted position. A yet further alternative is to locate a stop or rigid catching grid downstream of the diaphragm in order to limit excessive travel of the diaphragm as it snaps into its dynamic everted position, thus preventing burst, and also to maximize the rate of deceleration of the diaphragm to ensure release of the particles at the highest possible velocity. The stop or catching grid may have a similar profile to the diaphragm in its "fired" position and take the form of a shaped, perforated colander.

The shockwave that is propagated along the lumen, which is preferably in the form of a cylindrical shock tube, may be created by releasing compressed gas from the energizing means into a rupture chamber behind a rupturable membrane until the pressure difference across the membrane is sufficient to rupture the membrane and release the gas suddenly into the lumen. The driving gas is preferably lighter than the driven gas initially filling the shock tube, for example helium and air or carbon dioxide, respectively. This assists in enhancing the strength of the shockwave propagated in the shock tube, and hence increases the rate at which the diaphragm everts. In addition, the relative dimensions of the rupture chamber and the shock tube can be selected to provide for a significant contraction (area reduction) when passing from the rupture chamber to the shock tube, since this greatly enhances the strength of the shockwave, easily doubling its strength. Typically, the rupture chamber diameter is set at least 1.5 to 2 times greater than that of the shock tube. The rupturable membrane is formed from a suitable polymer material, for example polycarbonates (e.g., MACRAFOL^{™}), polyesters (e.g., MYLAR®) or other like materials. Both the membrane material and the thickness of the membrane material are selected to provide for a specific burst pressure.

The needleless syringe can further include a convergent cylindrical section interposed between the rupture chamber and the shock tube. The convergent cylindrical section has an upstream opening that is sized to correspond with the diameter of the rupture chamber, and a downstream opening that is sized to correspond with the reduced diameter of the shock tube, such that the section is convergent in the downstream direction. In preferred embodiments, the rupturable membrane is positioned over the downstream opening (narrower end) of the convergent cylindrical section in order to allow for a steady expansion of gas through the convergent section during release, and also to delay attenuation of the shockwave produced after rupture of the membrane, where such attenuation is due to the reflected expansion wave that passes back up through the shock tube. The convergent cylindrical section can be a simple molded annular part having a suitable convergent inner geometry.

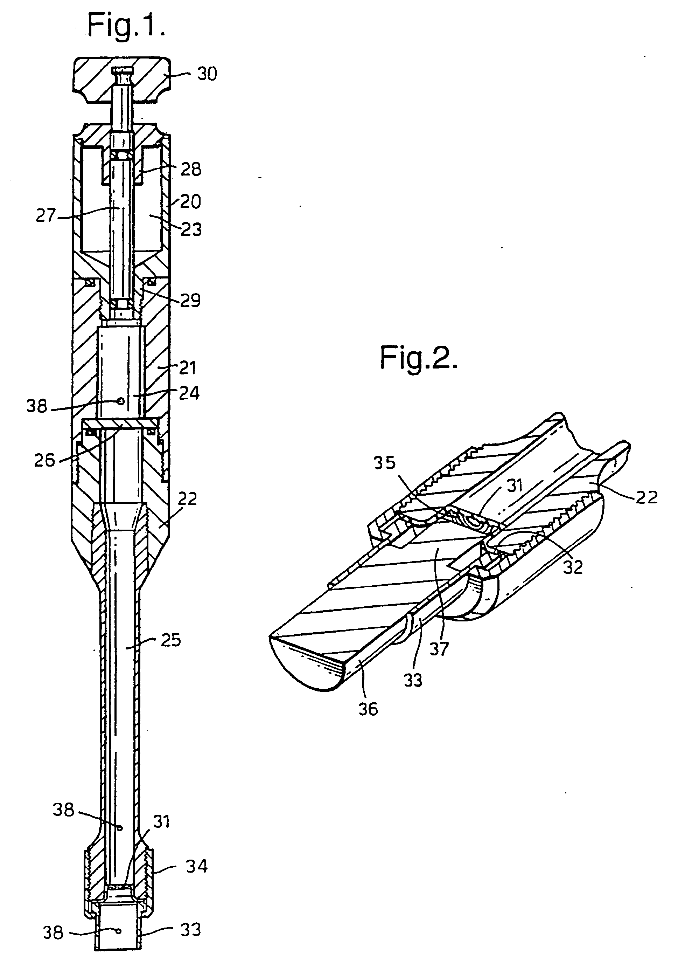

Referring now to the accompanying figures, a needleless syringe constructed according to the present invention is exemplified in Figure 1. The syringe of Figure 1 comprises three barrel portions **20, 21** and **22** which are connected and sealed together in axial alignment. The connection of these barrel portions can be by way of any suitable pressure-tight fit couplings and can further be held in place by pins, detents or other corresponding key- or snap-fit locking mechanisms. Alternatively, the barrel portions can be screwed together using corresponding threaded couplings. The upper barrel portion **20** provides a reservoir **23** which is initially filled with an energizing gas (e.g., helium or some other suitable gas) that is at a pressure on the order of about 20-80 bar, typically about 30 bar. The intermediate barrel portion **21** includes a rupture chamber **24**. The lower barrel portion **22** comprises an elongate body having a lumen extending therethrough. The upstream terminus of the lumen interfaces with the reservoir **23** by way of the rupture chamber **24**. Furthermore, the lumen extending through the lower barrel portion **22** includes an elongate shock tube **25**. Pinched and sealed around its edge between the upper and intermediate barrel portions **21** and **22** is a rupturable membrane **26** (shown of exaggerated thickness for clarity). The barrel portions are generally comprised of a suitably resilient material, for example, an injection-molded medical-grade polymer.

A valve stem **27** extends through the reservoir **23** and is slidable through bosses **28** and **29** to which it is initially sealed by 0-rings which it carries about its periphery. The end of the valve stem **27** which projects out of the top of reservoir **23** supports an operating button **30**.

A diaphragm **31** is positioned at the downstream terminus of the lumen which extends through the barrel portion **22** (the diaphragm **31** is shown as having a top hat section with a peripheral flange **32**). The flange is clamped between the lower end of the barrel portion **22** and the upper end of a tubular spacer **33** which is drawn up to the barrel portion by a gland nut **34.** The diaphragm therefore closes off the passage through the lumen formed by the shock tube **25**.

The external surface of the diaphragm comprises one or more topographical features for initially retaining particles. As can be seen in Figure 2, the particular topographical features in this device are in the form of a series of concentric annular ribs **35**, wherein the annular spaces between the concentric ribs form pockets for retaining particles on the surface of the diaphragm. As discussed above, an optional plug may be fitted in the concavity of the diaphragm to avoid displacement of the particles during transportation and handling prior to carrying out the particle delivery operation. One such plug **36**, which is push fit within the spacers **33** and has a reduced end portion **37** abutting the external surface of the diaphragm, is shown in Figure 2. The plug may be used to push and further retain the particles into the pockets of the diaphragm when the diaphragm is loaded and in its initial "pre-fired" position.

The modular construction of the syringe of Figures 1 and 2 allows for various transportation, storing and handling possibilities. For example, the barrel portion **20** can be stored and handled separately from the rest of the device components, and then readily fitted to the rest of the syringe immediately prior to use. In this regard, the barrel portions **21** and **22** will typically be separately packaged with the membrane **26** and diaphragm **31** in position; however, the barrel portions **21** and **22** are readily separable to allow the sandwiching between them of the optional rupturable membrane 26, and the spacer portion **33** is readily separable from the barrel portion **22** to allow the sandwiching between them of the diaphragm (which has an internal surface facing the lumen provided by the shock tube **25**, and an external surface facing outwardly relative to the syringe). The diaphragm **31** is moveable between an initial position in which a concavity is provided on the external surface of the diaphragm, and a dynamic position in which the external surface of the diaphragm is substantially convex. The barrel portions are also readily separable such that one or more of the syringe components can be provided as a disposable unit. Particles are initially provided in the concavity and selectively retained by the topographical features provided on the outwardly facing external surface of the diaphragm **31.**

In use, the needleless syringe of Figures 1 and 2 is assembled to provide suitable pressure-tight fittings between the components, and the shock tube **25** can optionally be pre-charged with a driven gas (for example air or carbon dioxide) at approximately atmospheric pressure (1 bar) or slightly higher (e.g., 2-3 bar). It is preferred, but not necessary, that the driven gas in the shock tube be heavier than the driving gas that is released from the energizing means. The plug **36** is removed, and the open end of the spacer **33** placed in proximity to, or in contact with, the target skin or mucosal surface to be treated. The button **30** is depressed, driving the valve stem **27** from its seat, thus releasing the pressurized driving gas from the reservoir **23** into the rupture chamber **24.** The maximum stroke of the stem **27** (before the button **30** abuts the end of the barrel portion **20**) is sufficient to allow the lower sealing ring to pass from its seat in the boss **29** into the rupture chamber **24** but not sufficient for the upper sealing ring to pass out of the boss **28** into the reservoir **23.** When the pressure in the rupture chamber **24** has reached a sufficient value the rupturable membrane **26** bursts, releasing a gaseous shockwave which propagates through the shock tube **25** and contacts the internal surface of the diaphragm **31.** The speed at which the reservoir **23** empties into the rupture chamber is not critical, but eventually the pressure in the rupture chamber **24** and the consequential differential pressure across the membrane **26** causes the membrane to rupture and to release a supersonic shock wave along the shock tube **25.** The impact of the gaseous shock wave upon the internal surface of the diaphragm provides sufficient force to suddenly impel the diaphragm from its initial position to a dynamic everted (outwardly convex) position, thereby dislodging the particles from the external surface of the diaphragm and propelling them toward the target surface. The particles are accelerated from the diaphragm at velocities sufficient for the transdermal delivery thereof across skin or mucosal tissue. A short tubular spacer **33** is provided to ensure that the diaphragm does not strike the target tissue, and to enable the particles to become more spread out and thus increase the effective target area. The spacer **33**, shock tube **25**, and/or rupture chamber **24** may each be provided with a vent hole **38** for releasing residual pressure from the device after firing.

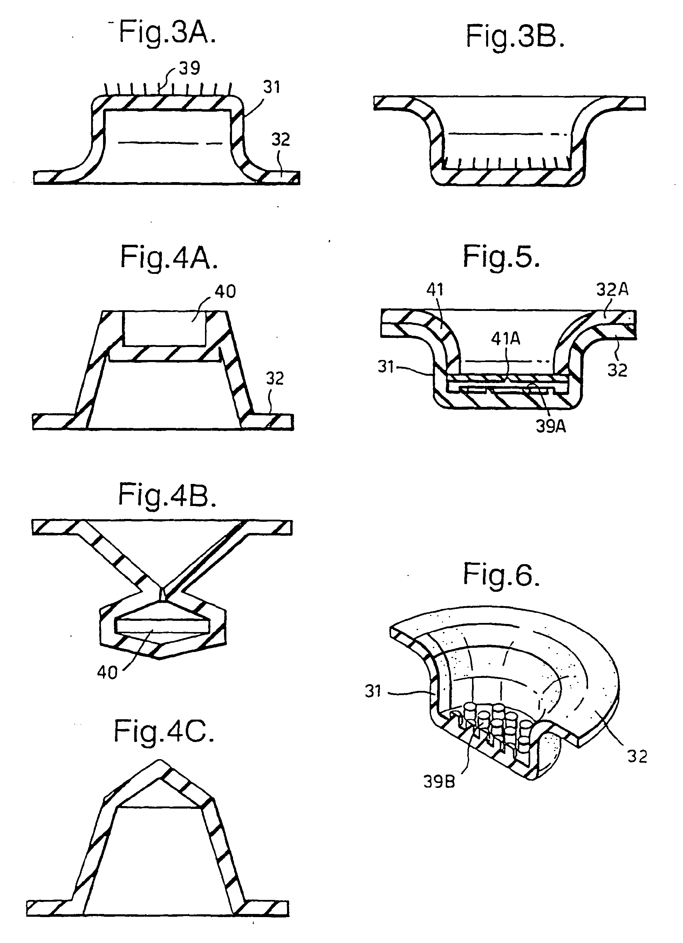

Referring now to Figures 3-12, various exemplary diaphragm constructions are depicted. For example, Figures 3A-3B show a diaphragm **31** having an overall configuration in the shape of a top hat. Figure 3A shows the diaphragm in its "start," i.e., as molded, and "fired" position. The annular peripheral flange **32** provides a lip that can be clamped between two parts of a needleless syringe (e.g., between portions **25** and **33** of the device of Figures 1-2), and the diaphragm is thus mounted in a syringe as described with reference to Figures 1 and 2. On its substantially flat, external surface, the diaphragm is integrally molded with a plurality of bristles **39** which are increasingly radially outwardly divergent towards the edge. After the particles have been located on the external surface, about the bristles, the diaphragm is gently inverted into the "loaded" position as shown in Figure 3B. As can be seen, this causes the bristles to converge radially inwardly to an increasing extent towards the edge of the array. The particles are thus held to a significant extent by the disposition of the bristles until the diaphragm is suddenly everted to the "fired" position whereupon the particles are catapulted outwards.

Figures 4A-4C show a more complicated diaphragm configuration, of which Figure 4A shows the diaphragm in its "start" position with an open pocket **40** ready to be charged with particles. Figure 4B shows the "loaded" position in which the pocket **40** is substantially completely closed, and Figure 4C shows the "fired" position. In this embodiment, the topographical feature (i.e., the pocket **40**) comprises both the recessed pocket and surrounding kinks or folds which allow the diaphragm to fold back on itself in the loaded position and closely retain the particles.

Figure 5 shows a diaphragm **31** in its initial, "loaded" position and having an array of bristles **39A** depending and extending from the external surface of the diaphragm. The particles, which are contained within the pocket provided by the topographical features are further initially retained by a plug which is in the form of a stopper **41** and is of similar shape to the diaphragm **31.** This allows for a tight fit of the stopper within the diaphragm. In addition, the stopper **41** has a peripheral flange **32A** which corresponds with the peripheral flange of the diaphragm and thus can be retained in the downstream end of the shock tube in the same way as the diaphragm in Figures 1 and 2. The substantially flat central portion of the top hat section of the stopper **41** is provided with one or more lines of weakness **41A** that allow this stopper to break apart and readily move with the diaphragm as it everts, thus substantially not impeding the catapulting of the particles from the exterior surface of the diaphragm. The stopper **41** therefore acts as an alternative to the plug **36** shown in Figure 2 and does not have to be handled prior to use of the syringe.

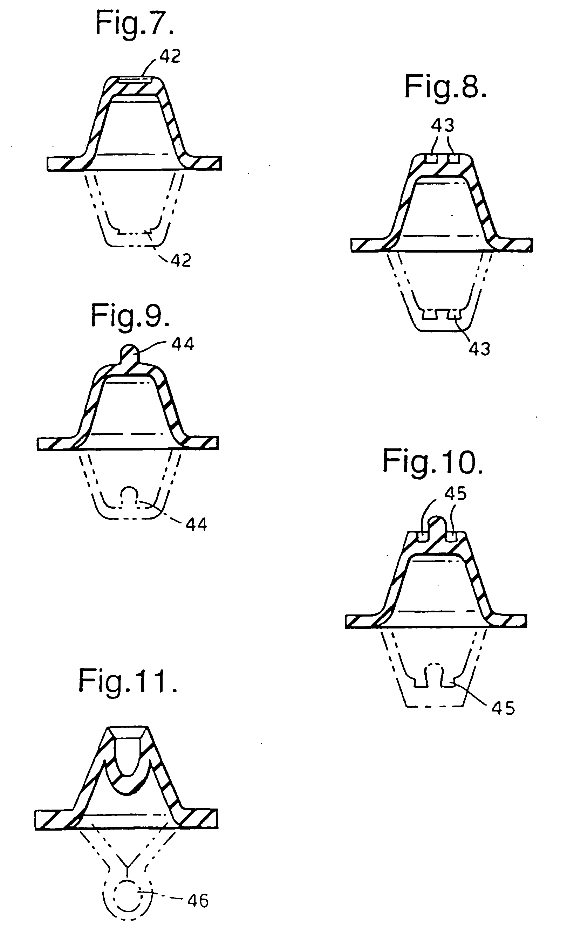

Figure 6 shows schematically, and in its initial, "loaded" position, a modification of the diaphragm of Figure 3, in which the bristles **39B** are more robust and stubby than those indicated in Figure 3. Figures 7-11 show still further alternative diaphragm configurations comprising topographical features on their external surfaces, where each diaphragm is shown in its molded and dynamic "fired" position in full lines, and in their initial, "loaded" position in chain dotted lines. Whereas the diaphragms of Figures 3-6 are typically circularly symmetrical, it is possible for the diaphragms of Figures 7-11 to be somewhat elongate with the section being taken across the narrow dimension.

In particular, the diaphragm depicted in Figure 7 has a centrally disposed hollow **42** that forms a pocket for selectively retaining particles on the external surface of the diaphragm when it is in its initial, "loaded" position. The diaphragm of Figure 8 comprises topographical features that form one or more pockets, wherein the particular features are either a single groove **43** (e.g., annular) or a plurality of groove(s). The diaphragm of Figure 9 contains a finger projection or rib **44** that depends from the external surface of the diaphragm and extends outwardly therefrom. The diaphragm of Figure 10 is similar to that of Figure 9, except that it has, on each side of the finger or rib **44**, one or more grooves **45** (e.g., when the diaphragm includes a finger **44**, a single annular groove **45** is used to establish a pocket in combination with the finger, and when the diaphragm includes a rib **44,** a plurality of grooves **45** are used to establish a plurality of pockets in combination with the central rib). Finally, the diaphragm of Figure 11 comprises kinks or folds which serve to establish a pocket **46** which is substantially closed while the diaphragm is in its initial "loaded" position.

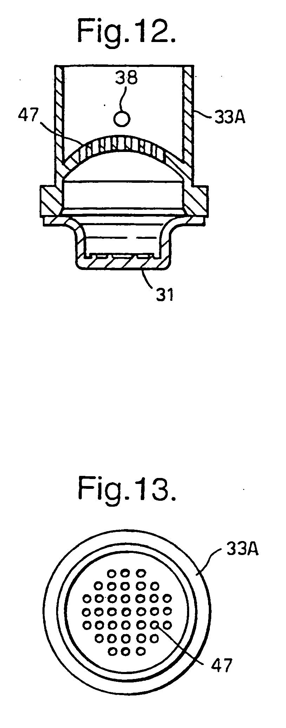

Figures 12 and 13 show a modified spacer **33A** containing an integral perforated catching grid **47** which, as the syringe is fired and the diaphragm **31** everts, prevents over-extension and possible bursting of the diaphragm, whilst the holes in the grid substantially do not impede the catapulted particles.

In accordance with another embodiment of the present invention, a needleless syringe having enhanced particle spread and/or particle payload capacity is provided. This needleless syringe includes a body having a lumen passing therethrough, wherein the lumen has an upstream terminus and a downstream terminus and the upstream terminus of the lumen is capable of interfacing with an energizing means. Typically all of the components of the device of Figures 1 and 2 are included, with the difference being that a plurality of diaphragms are arranged adjacent to the downstream terminus of the lumen. Each of these diaphragms have an internal surface facing the lumen and an external surface, and the diaphragms are moveable between an initial position in which a concavity is provided on their external surfaces, and a dynamic position in which the external surface of the diaphragms is substantially convex. By using multiple diaphragms, the target spread and/or total possible particle payload are significantly increased.

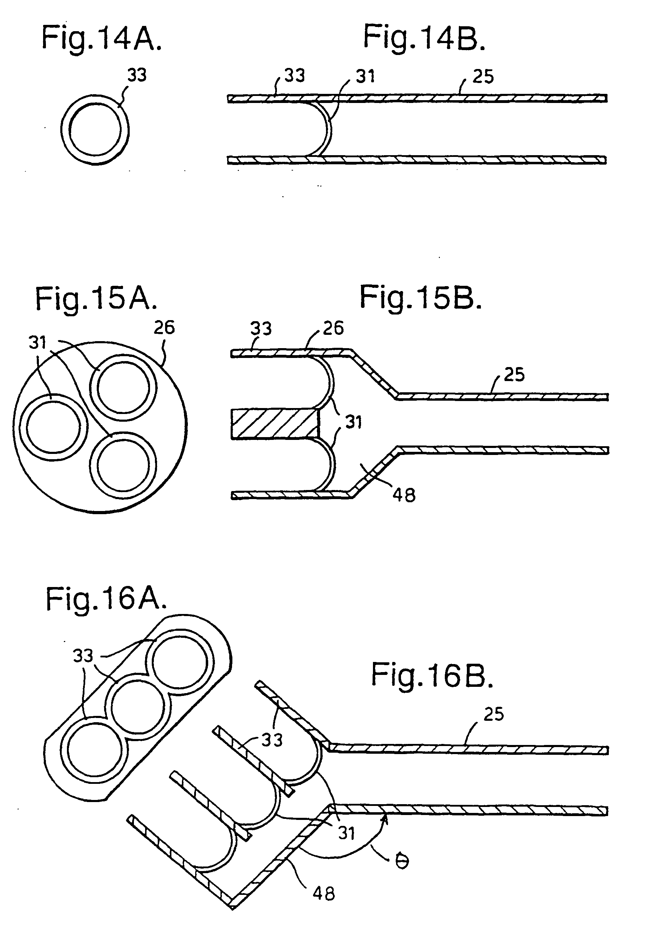

Referring now to Figures 14-16, the downstream portions of various devices constructed according to the present invention are shown. For example, Figure 14 shows diagrammatically a shock tube **25** containing, in axial alignment, a single diaphragm **31** and spacer **33**, similarly to the configuration of the device of Figures 1-2. However, Figures 15A-15B show a modification in which the shock tube **25** opens out into a delivery head **48** which houses a plurality (three) diaphragms **31** that are arranged in a circular array. In this configuration, a single shock wave propagated through the shock tube is used to evert all three diaphragms simultaneously, and thus deliver a triple payload of particles over a larger target surface area. As will be understood by the ordinarily skilled artisan upon reading the instant specification, this device set-up can be used to simultaneously deliver two or more different particle compositions, for example where one is desirous of delivering a pattern of contact allergens in a dermal skin test, or can be used to broaden particle distribution and increase payload of a single particle composition. In a related embodiment, as depicted in Figures 16A-16B, the multiple diaphragms **31** can be arranged in a linear array. In addition, the delivery head can be aligned with the central axis of the shock tube **25** over an angle θ which can range from 90-180°.

The multiple-diaphragm head devices of Figures 15-16 can be fitted with standard, dome-shaped diaphragms, or with diaphragms which provide for enhanced particle retention as described in the present invention. Furthermore, the various positioning of rupturable membranes, use of convergent cylindrical sections, and relative rupture chamber/shock tube dimensions described in detail above with respect to the single-diaphragm devices of the present invention can be used with these multiple-diaphragm devices in order to enhance or optimize their delivery performance.

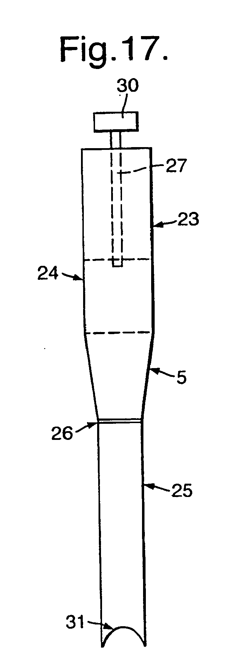

In accordance with a still further embodiment of the present invention, a high-powered needleless syringe is provided. The needleless syringe has, in operative combination, (a) a body with a lumen passing therethrough, (b) a rupture chamber having an upstream opening and a downstream opening, wherein the upstream opening of the rupture chamber is adapted to interface with an energizing means, (c) a convergent cylindrical section interposed between the rupture chamber and the lumen, wherein the convergent cylindrical section has an upstream opening that is in fluid communication with the downstream opening of the rupture chamber, and a downstream opening that is in fluid communication with the upstream terminus of the lumen, and further wherein the downstream opening of said convergent cylindrical section is closed by a rupturable membrane, and (d) a diaphragm arranged adjacent to the downstream terminus of the lumen. A diagram of the positional relationship among the components of the subject device embodiment is shown in Figure 17, wherein the diaphragm **31**, the lumen containing the shock tube **25**, the rupturable membrane **26**, the convergent cylindrical section **5**, the rupture chamber 24, energizing means **23**, piston **27**, and button **30** are as substantially as described herein above with respect to the other device embodiments of the present invention.

As with the above-described devices, the diaphragm has an internal surface facing the lumen and an external surface, wherein the diaphragm is moveable between an initial position in which a concavity is provided on the external surface of the diaphragm, and a dynamic position in which the external surface of the diaphragm is substantially convex. The needleless syringe is assembled and operated in the same manner as the other embodiments of the invention, and provides a shockwave energizing force that is about twice as powerful as previous such devices.

Each of the needleless syringes of the present invention can be used for transdermal delivery of powdered therapeutic compounds and compositions, for delivery of genetic material into living cells (e.g., gene therapy or nucleic acid vaccination), both *in vivo* and *ex vivo,* and for the delivery of biopharmaceuticals to skin, muscle, blood or lymph. The syringes can also be used in conjunction with surgery to deliver therapeutic agents, drugs, immunogens, and/or biologies to organ surfaces, solid tumors and/or to surgical cavities (e.g., tumor beds or cavities after tumor resection). Further, the instant devices can be used in the transdermal delivery of generally inert particles for the purpose of non- or minimally invasive sampling of an analyte from a biological system. In theory, practically any agent that can be prepared in a substantially solid, particulate form can be safely and easily delivered using the present devices.

Delivery of particles from the above-described needleless syringe systems is generally practiced using particles having an approximate size generally ranging from 0.1 to 250 µm. For drug delivery, the optimal particle size is usually at least about 10 to 15 µm (the size of a typical cell). For gene delivery, the optimal particle size is generally substantially smaller than 10 µm. Particles larger than about 250 µm can also be delivered from the devices, with the upper limitation being the point at which the size of the particles would cause untoward damage to the skin cells. The actual distance which the delivered particles will penetrate a target surface depends upon particle size (e.g., the nominal particle diameter assuming a roughly spherical particle geometry), particle density, the initial velocity at which the particle impacts the surface, and the density and kinematic viscosity of the targeted skin or mucosal tissue. In this regard, optimal particle densities for use in needleless injection generally range between about 0.1 and 25 g/cm³, preferably between about 0.9 and 1.5 g/cm³, and injection velocities can range from about 150 to about 3,000 m/sec.

Accordingly, novel needleless syringe delivery systems and methods for using the same are disclosed. Although preferred embodiments of the subject invention have been described in some detail, it is understood that obvious variations can be made without departing from the scope of the invention as defined by the appended claims.

## Claims

1. A needleless syringe for accelerating particles into a target tissue surface of a vertebrate subject, said syringe comprising:
(a) a body (25) having a lumen therein, wherein said lumen has an upstream terminus and a downstream terminus and the upstream terminus of the lumen is capable of interfacing with an energizing means (23); and
(b) a diaphragm (31) arranged adjacent to the downstream terminus of the lumen, said diaphragm (31) having an internal surface facing the lumen and an external surface, wherein said diaphragm (31) is moveable between an initial position in which a concavity is provided on the external surface of the diaphragm, and a dynamic position in which the external surface of the diaphragm is substantially convex, wherein the external surface of said diaphragm (31) comprises one or more topographical features (39) which selectively retain particles on the external surface of the diaphragm (31) when in its initial position; and **characterized in that** the diaphragm (31) has an overall configuration in the shape of a top hat.

2. The syringe of claim 1 wherein said one or more topographical features on the external surface of the diaphragm comprise a pocket (40) for retaining particles.

3. The syringe of claim 2 wherein the pocket (40) is provided by a groove (43), channel or trough in the external surface of the diaphragm (31).

4. The syringe of claim 2 wherein the pocket (40) is provided by one or more superficial cuts or scores on the external surface of the diaphragm (31).

5. The syringe of claim 2 wherein the pocket (40) is provided by a fold or kink in the diaphragm.

6. The syringe of claim 1 wherein said one or more topographical features on the external surface of the diaphragm (31) comprise a structure (44) depending and extending outwardly from the external surface of said diaphragm.

7. The syringe of claim 6 wherein the external surface of the diaphragm comprises one or more bristles (39) depending from the diaphragm (31) and extending outwardly therefrom.

8. The syringe of claim 7 wherein the external surface of the diaphragm (31) comprises one or more ribs (44), fins or structural partitions depending from the diaphragm (31) and extending outwardly therefrom.

9. The syringe of claim 7 wherein the external surface of the diaphragm (31) comprises one or more annular ribs, fins or structural partitions depending from the diaphragm and extending outwardly therefrom.

10. The syringe of any one of claims 1-9 wherein the diaphragm (31) is reinforced with a fabric attached or adhered to the internal or external surface of the diaphragm.

11. The syringe of any one of claims 1-10 further comprising a stop (47) arranged at or over the downstream terminus of the lumen such that said stop prevents excess travel of the diaphragm (31) as it moves to its dynamic, outwardly convex position.

12. The syringe of any one of claims 1-11 wherein the lumen comprises an elongate cylindrical shock tube (25).

13. The syringe of claim 12 wherein the shock tube (25) comprises an aperture (38) for venting gases therefrom.

14. The syringe of claim 12 further comprising a rupture chamber (24) having a first opening adapted to interface with an energizing means (23), and a second opening which is in fluid communication with the upstream terminus of the lumen.

15. The syringe of claim 14, wherein the second opening of the rupture chamber is closed by a rupturable membrane (26).

16. The syringe of claim 12 further comprising:
(a) a rupture chamber (24) having an upstream opening and a downstream opening, wherein the upstream opening of said rupture chamber is adapted to interface with an energizing means (23); and
(b) a convergent cylindrical section (5) interposed between the rupture chamber (24) and the lumen (25), wherein said convergent cylindrical (5) section has an upstream opening that is in fluid communication with the downstream opening of the rupture chamber (24), and a downstream opening that is in fluid communication with the upstream terminus of the lumen, and further wherein the downstream opening of said convergent cylindrical section (5) is closed by a rupturable membrane (26).

17. The syringe of claim 16 wherein the rupture chamber (24) has a diameter which is at least about 1.5 times greater than that of the shock tube (25).

18. The syringe of any one of claims 1-17 wherein the external surface of the diaphragm (31) is tacky.

19. A needleless syringe for accelerating particles into a target tissue surface of a vertebrate subject, said syringe comprising:
(a) a body (25) having a lumen therein, wherein said lumen has an upstream terminus and a downstream terminus and the upstream terminus of the lumen is capable of interfacing with an energizing means (23); and .
(b) a plurality of diaphragms (31) having an overall configuration in the shape of a top hat, arranged adjacent to the downstream terminus of the lumen, each said diaphragm (31) having an internal surface facing the lumen and an external surface, wherein said diaphragms (31) are moveable between an initial position in which a concavity is provided on the external surface of said diaphragms, and a dynamic position in which the external surface of said diaphragms is substantially convex.

20. The syringe of claim 19, wherein the plurality of diaphragms (31) are arranged in an array.

21. The syringe of claim 20 wherein the plurality of diaphragms (31) are arranged in a linear array.

22. The syringe of any one of claims 19-21 wherein the downstream terminus of the lumen widens into a chamber behind the upstream surfaces of said plurality of diaphragms (31).

23. The syringe of any one of claims 19-22 wherein a central region of the external surface of at least one of said diaphragms (31) is substantially flat or planar.

24. The syringe of any one of claims 19-23 wherein the external surface of at least one of said diaphragms (31) comprises one or more topographical features (39) that selectively retain particles on the external surface of said diaphragm (31) when in its initial position.

25. The syringe of claim 24 wherein the one or more topographical features on the external surface of the diaphragm comprise a pocket (40) for retaining particles.

26. The syringe of claim 25 wherein said one or more topographical features on the external surface of the diaphragm comprise a structure (44) depending and extending outwardly from the external surface of said diaphragm.

27. The syringe of any one of claims 19-26 wherein the lumen comprises an elongate cylindrical shock tube (25).

28. The syringe of claim 27 wherein the shock tube (25) comprises an aperture (38) for venting gases therefrom.

29. The syringe of claim 27 further comprising a rupture chamber (24) having a first opening adapted to interface with an energizing means (23), and a second opening which is in fluid communication with the upstream terminus of the lumen.

30. The syringe of claim 29, wherein the second opening of the rupture chamber (24) is closed by a rupturable membrane (26).

31. The syringe of claim 27 further comprising:
(a) a rupture chamber (24) having an upstream opening and a downstream opening, wherein the upstream opening of said rupture chamber (24) is adapted to interface with an energizing means (23); and
(b) a convergent cylindrical section (5) interposed between the rupture chamber (24) and the lumen, wherein said convergent cylindrical section (5) has an upstream opening that is in fluid communication with the downstream opening of the rupture chamber (24), and a downstream opening that is in fluid communication with the upstream terminus of the lumen, and further wherein the downstream opening of said convergent cylindrical section (5) is closed by a rupturable membrane (26).

32. The syringe of any one of claims 29-31 wherein the rupture chamber (24) has a diameter which is at least about 1.5 times greater than that of the shock tube (25).

33. A needleless syringe for accelerating particles into a target tissue surface of a vertebrate subject, said syringe comprising:
(a) a body (25) having a lumen therein, wherein said lumen has an upstream terminus and a downstream terminus;
(b) a rupture chamber (24) having an upstream opening and a downstream opening, wherein the upstream opening of said rupture chamber is adapted to interface with an energizing means (23);
(c) a convergent cylindrical section (5) interposed between the rupture chamber (24) and the lumen, wherein said convergent cylindrical section (5) has an upstream opening that is in fluid communication with the downstream opening of the rupture chamber (24), and a downstream opening that is in fluid communication with the upstream terminus of the lumen, and further wherein the downstream opening of said convergent cylindrical section (5) is closed by a rupturable membrane (26); and
(d) a diaphragm (31) arranged adjacent to the downstream terminus of the lumen, said diaphragm (31) having an internal surface facing the lumen and an external surface, wherein said diaphragm (31) is moveable between an initial position in which a concavity is provided on the external surface of the diaphragm (31), and a dynamic position in which the external surface of the diaphragm (31) is substantially convex, wherein said diaphragm (31) has an overall configuration in the shape of a top hat.

34. The syringe of claim 33 wherein the lumen comprises an elongate cylindrical shock tube (25).

35. The syringe of claim 34 wherein the rupture chamber (24) has a diameter which is at least about 1.5 times greater than that of the shock tube (25).

## Patentansprüche

1. Nadellose Spritze zum Beschleunigen von Partikeln in eine Zielgewebefläche eines eine Wirbelsäule aufweisenden Patienten, wobei die Spritze umfasst:
a) einen Körper (25) mit einem Lumen darin, wobei das Lumen ein stromaufwärts befindliches Ende und ein stromabwärts befindliches Ende aufweist und das stromaufwärts befindliche Ende des Lumen mit einem Energie zuführenden Mittel (23) koppeln kann; und
(b) eine neben dem stromabwärts befindlichen Ende des Lumen angeordnete Membran (31), wobei die Membran (31) eine dem Lumen zugewandte Innenfläche und eine Außenfläche aufweist, wobei die Membran (31) zwischen einer anfänglichen Position, bei der an der Außenfläche der Membran eine Höhlung vorgesehen ist, und einer dynamischen Position, bei der die Außenfläche der Membran im Wesentlichen konvex ist, beweglich ist, wobei die Außenfläche der Membran (31) ein oder mehrere topographische Merkmale (39) aufweist, die Partikel wahlweise auf der Außenfläche der Membran (31) zurückhalten, wenn sich diese in der anfänglichen Position befindet; und **dadurch gekennzeichnet, dass** die Membran (31) insgesamt eine Konfiguration in Form eines Zylinderhuts hat.

2. Spritze nach Anspruch 1, **dadurch gekennzeichnet, dass** das eine oder die mehreren topographischen Merkmale an der Außenfläche der Membran eine Tasche (40) zum Zurückhalten von Partikeln umfassen.

3. Spritze nach Anspruch 2, **dadurch gekennzeichnet, dass** die Tasche (40) durch eine Nut (43), einen Kanal oder eine Mulde in der Außenfläche der Membran (31) vorgesehen wird.

4. Spritze nach Anspruch 2, **dadurch gekennzeichnet, dass** die Tasche (40) durch einen oder mehrere oberflächliche Schnitte oder Rillen an der Außenfläche der Membran (31) vorgesehen wird.

5. Spritze nach Anspruch 2, **dadurch gekennzeichnet, dass** die Tasche (40) durch eine Falte oder einen Knick in der Membran vorgesehen wird.

6. Spritze nach Anspruch 1, **dadurch gekennzeichnet, dass** ein oder mehrere topographische Merkmale an der Außenfläche der Membran (31) eine Struktur (44) aufweisen, die von der Außenfläche der Membran abhängt und sich nach außen erstreckt.

7. Spritze nach Anspruch 6, **dadurch gekennzeichnet, dass** die Außenfläche der Membran eine oder mehrere Borsten (39) umfasst, die von der Membran (31) abhängen und sich davon nach außen erstrecken.

8. Spritze nach Anspruch 7, **dadurch gekennzeichnet, dass** die Außenfläche der Membran (31) eine oder mehrere Rippen (44), Grate oder strukturelle Abtrennungen umfasst, die von der Membran (31) abhängen und sich davon nach außen erstrecken.

9. Spritze nach Anspruch 7, **dadurch gekennzeichnet, dass** die Außenfläche der Membran (31) eine oder mehrere ringförmige Rippen, Grate oder strukturelle Abtrennungen umfasst, die von der Membran abhängen und sich davon nach außen erstrecken.

10. Spritze nach einem der Ansprüche 1-9, **dadurch gekennzeichnet, dass** die Membran (31) mit einem Gewebe verstärkt ist, das an der Innen- oder Außenfläche der Membran angebracht ist oder an dieser anhaftet.

11. Spritze nach einem der Ansprüche 1-10, welche weiterhin einen Anschlag (47) umfasst, der an oder über dem stromabwärts befindlichen Ende des Lumen angeordnet ist, so dass der Anschlag einen Überlauf der Membran (31) verhindert, wenn sie sich zu ihrer dynamischen, nach außen konvexen Position bewegt.

12. Spritze nach einem der Ansprüche 1-11, **dadurch gekennzeichnet, dass** das Lumen ein längliches zylinderförmiges Stoßwellenrohr (25) umfasst.

13. Spritze nach Anspruch 12, **dadurch gekennzeichnet, dass** das Stoßwellenrohr (25) eine Öffnung (38) zum Ablassen von Gasen daraus umfasst.

14. Spritze nach Anspruch 12, welche weiterhin eine Berstkammer (24) mit einer dafür ausgelegten ersten Öffnung, mit einem Energie zuführenden Mittel (23) zu koppeln, und mit einer zweiten Öffnung, die mit dem stromaufwärts befindlichen Ende des Lumen in Fluidverbindung steht, umfasst.

15. Spritze nach Anspruch 14, **dadurch gekennzeichnet, dass** die zweite Öffnung der Berstkammer durch eine berstbare Membran (26) verschlossen ist,

16. Spritze nach Anspruch 12, welche weiterhin umfasst:
(a) eine Berstkammer (24) mit einer stromaufwärts befindlichen Öffnung und einer stromabwärts befindlichen Öffnung, wobei die stromaufwärts befindliche Öffnung der Berstkammer dafür ausgelegt ist, mit einem Energie zuführenden Mittel (23) zu koppeln; und
(b) einen zwischen der Berstkammer (24) und dem Lumen (25) dazwischen angeordneten konvergenten zylindrischen Abschnitt (5), wobei der konvergente zylindrische Abschnitt (5) eine stromaufwärts befindliche Öffnung, die in Fluidverbindung mit der stromabwärts befindlichen Öffnung der Berstkammer (24) steht, und eine stromabwärts befindliche Öffnung, die in Fluidverbindung mit dem stromaufwärts befindlichen Ende des Lumen steht, aufweist und wobei weiterhin die stromabwärts befindliche Öffnung des konvergenten zylindrischen Abschnitts (5) durch eine berstbare Membran (26) verschlossen ist.

17. Spritze nach Anspruch 16, **dadurch gekennzeichnet, dass** die Berstkammer (24) einen Durchmesser aufweist, der mindestes um das etwa 1,5fache größer als der des Stoßwellenrohrs (25) ist,

18. Spritze nach einem der Ansprüche 1-17, **dadurch gekennzeichnet, dass** die Außenfläche der Membran (31) klebrig ist.

19. Nadellose Spritze zum Beschleunigen von Partikeln in eine Zielgewebefläche eines eine Wirbelsäule aufweisenden Patienten, wobei die Spritze umfasst:
a) einen Körper (25) mit einem Lumen darin, wobei das Lumen ein stromaufwärts befindliches Ende und ein stromabwärts befindliches Ende aufweist und das stromaufwärts befindliche Ende des Lumen mit einem Energie zuführenden Mittel (23) koppeln kann; und
(b) mehrere Membranen (31) mit einer Gesamtkonfiguration in Form eines Zylinderhuts, die neben dem stromabwärts befindlichen Ende des Lumen angeordnet sind, wobei jede Membran (31) eine dem Lumen zugewandte Innenfläche und eine Außenfläche aufweist, wobei die Membranen (31) zwischen einer anfänglichen Position, bei der an der Außenfläche der Membranen eine Höhlung vorgesehen ist, und einer dynamischen Position, bei der die Außenfläche der Membranen Im Wesentlichen konvex ist, beweglich sind.

20. Spritze nach Anspruch 19, **dadurch gekennzeichnet, dass** die mehreren Membranen (31) in einer Anordnung angeordnet sind.

21. Spritze nach Anspruch 20, **dadurch gekennzeichnet, dass** die mehreren Membranen (31) in einer geradlinigen Anordnung angeordnet sind.

22. Spritze nach einem der Ansprüche 19-21, **dadurch gekennzeichnet, dass** sich das stromabwärts befindliche Ende des Lumen zu einer Kammer hinter den stromaufwärts befindlichen Flächen der mehreren Membranen (31) weitet.

23. Spritze nach einem der Ansprüche 19-22, **dadurch gekennzeichnet, dass** ein mittlerer Bereich der Außenfläche mindestens einer der Membranen (31) im Wesentlichen flach oder eben ist.

24. Spritze nach einem der Ansprüche 19-23, **dadurch gekennzeichnet, dass** die Außenfläche mindestens einer der Membranen (31) ein oder mehrere topographische Merkmale (39) umfasst, die Partikel wahlweise an der Außenfläche der Membran (31) festhalten, wenn sich diese in ihrer anfänglichen Position befindet.

25. Spritze nach Anspruch 24, **dadurch gekennzeichnet, dass** das eine oder die mehreren topographischen Merkmale an der Außenfläche der Membran eine Tasche (40) zum Rückhalten von Partikeln umfassen.

26. Spritze nach Anspruch 25, **dadurch gekennzeichnet, dass** das eine oder die mehreren topographischen Merkmale an der Außenfläche der Membran eine Struktur (44) aufweisen, die von der Außenfläche der Membran abhängt und sich nach außen erstreckt.

27. Spritze nach einem der Ansprüche 19-26, **dadurch gekennzeichnet, dass** das Lumen ein längliches zylinderförmiges Stoßwellenrohr (25) umfasst.

28. Spritze nach Anspruch 27, **dadurch gekennzeichnet, dass** das Stoßwellenrohr (25) eine Öffnung (38) zum Ablassen von Gasen daraus umfasst.

29. Spritze nach Anspruch 27, welche weiterhin eine Berstkammer (24) mit einer dafür ausgelegten ersten Öffnung, mit einem Energie zuführenden Mittel (23) zu koppeln, und mit einer zweiten Öffnung, die mit dem stromaufwärts befindlichen Ende des Lumen in Fluidverbindung steht, umfasst,

30. Spritze nach Anspruch 29, **dadurch gekennzeichnet, dass** die zweite Öffnung der Berstkammer (24) durch eine berstbare Membran (26) verschlossen ist.

31. Spritze nach Anspruch 27, welche weiterhin umfasst:
(a) eine Berstkammer (24) mit einer stromaufwärts befindlichen Öffnung und einer stromabwärts befindlichen Öffnung, wobei die stromaufwärts befindliche Öffnung der Berstkammer (24) dafür ausgelegt ist, mit einem Energie zuführenden Mittel (23) zu koppeln; und
(b) einen zwischen der Berstkammer (24) und dem Lumen (25) dazwischen angeordneten konvergenten zylindrischen Abschnitt (5), wobei der konvergente zylindrische Abschnitt (5) eine stromaufwärts befindliche Öffnung, die in Fluidverbindung mit der stromabwärts befindlichen Öffnung der Berstkammer (24) steht, und eine stromabwärts befindliche Öffnung, die in Fluidverbindung mit dem stromaufwärts befindlichen Ende des Lumen steht, aufweist und wobei weiterhin die stromabwärts befindliche Öffnung des konvergenten zylindrischen Abschnitts (5) durch eine berstbare Membran (26) verschlossen ist.

32. Spritze nach einem der Ansprüche 29-31, **dadurch gekennzeichnet, dass** die Berstkammer (24) einen Durchmesser aufweist, der mindestes um das etwa 1,5fache größer als der des Stoßwellenrohrs (25) ist.

33. Nadellose Spritze zum Beschleunigen von Partikeln in eine Zielgewebefläche eines eine Wirbelsäule aufweisenden Patienten, wobei die Spritze umfasst:
(a) einen Körper (25) mit einem Lumen darin, wobei das Lumen ein stromaufwärts befindliches Ende und ein stromabwärts befindliches Ende aufweist;
(b) eine Berstkammer (24) mit einer stromaufwärts befindlichen Öffnung und einer stromabwärts befindlichen Öffnung, wobei die stromaufwärts befindliche Öffnung der Berstkammer dafür ausgelegt ist, mit einem Energie zuführenden Mittel (23) zu koppeln;
(c) einen zwischen der Berstkammer (24) und dem Lumen (25) dazwischen angeordneten konvergenten zylindrischen Abschnitt (5), wobei der konvergente zylindrische Abschnitt (5) eine stromaufwärts befindliche Öffnung, die in Fluidverbindung mit der stromabwärts befindlichen Öffnung der Berstkammer (24) steht, und eine stromabwärts befindliche Öffnung, die in Fluidverbindung mit dem stromaufwärts befindlichen Ende des Lumen steht, aufweist und wobei weiterhin die stromabwärts befindliche Öffnung des konvergenten zylindrischen Abschnitts (5) durch eine berstbare Membran (26) verschlossen ist; und
(d) eine neben dem stromabwärts befindlichen Ende des Lumen angeordnete Membran (31), wobei die Membran (31) eine dem Lumen zugewandte Innenfläche und eine Außenfläche aufweist, wobei die Membran (31) zwischen einer anfänglichen Position, bei der an der Außenfläche der Membran (31) eine Höhlung vorgesehen ist, und einer dynamischen Position, bei der die Außenfläche der Membran (31) in Wesentlichen konvex ist, beweglich ist; wobei die Membran (31) eine Gosamtkonfiguration in Form eines Zylinderhuts hat.

34. Spritze nach Anspruch 33, **dadurch gekennzeichnet, dass** das Lumen ein längliches zylinderförmiges Stoßwellenrohr (25) umfasst.

35. Spritze nach Anspruch 34, **dadurch gekennzeichnet, dass** die Berstkammer (24) einen Durchmesser aufweist, der mindestes um das etwa 1,5fache größer als der des Stoßwellenrohrs (25) ist.

## Revendications

1. Seringue sans aiguille pour provoquer une accélération de particules vers une surface tissulaire visée d'un sujet vertébré, ladite seringue comprenant :
(a) un corps (25) comportant un conduit, ledit conduit ayant une extrémité amont et une extrémité aval et l'extrémité amont du conduit pouvant être en interface avec un moyen d'excitation (23) ; et
(b) un diaphragme (31) disposé au voisinage immédiat de l'extrémité aval du conduit, ledit diaphragme (31) ayant une surface intérieure en regard du conduit et une surface extérieure, ledit diaphragme (31) étant mobile entre une position initiale dans laquelle un espace concave est créé sur la surface extérieure du diaphragme, et une position dynamique dans laquelle la surface extérieure du diaphragme est sensiblement convexe, la surface extérieure dudit diaphragme (31) comportant un ou plusieurs reliefs (39) qui retiennent de manière sélective des particules sur la surface extérieure du diaphragme (31) lorsqu'il est dans sa position initiale ; et **caractérisé en ce que** le diaphragme (31) a globalement l'aspect d'un chapeau haut de forme.

2. Seringue selon la revendication 1, dans laquelle un ou plusieurs reliefs sur la surface extérieure du diaphragme sont constitués par une poche (40) destinée à retenir des particules.

3. Seringue selon la revendication 2, dans laquelle la poche (40) est constituée par une gorge (43), un canal ou une dépression dans la surface extérieure du diaphragme (31).

4. Seringue selon la revendication 2, dans laquelle la poche (40) est constituée par une ou plusieurs entailles ou encoches dans la surface extérieure du diaphragme (31).

5. Seringue selon la revendication 2, dans laquelle la poche (40) est constituée par un pli ou une pliure dans le diaphragme.

6. Seringue selon la revendication 1, dans laquelle ledit ou lesdits reliefs sur la surface extérieure du diaphragme (31) constituent une structure (44) suspendue à et s'étendant vers l'extérieur depuis la surface extérieure dudit diaphragme.

7. Seringue selon la revendication 6, dans laquelle la surface extérieure du diaphragme comporte une ou plusieurs soies (39) suspendues au diaphragme (31) et s'étendant vers l'extérieur depuis celui-ci.

8. Seringue selon la revendication 7, dans laquelle la surface extérieure du diaphragme (31) comporte une ou plusieurs nervures (44), ailettes ou cloisons suspendues au diaphragme (31) et s'étendant vers l'extérieur depuis celui-ci.

9. Seringue selon la revendication 7, dans laquelle la surface extérieure du diaphragme (31) comporte une ou plusieurs nervures, ailettes ou cloisons suspendues au diaphragme et s'étendant vers l'extérieur depuis celui-ci.

10. Seringue selon l'une quelconque des revendications 1 à 9, dans laquelle le diaphragme (31) est renforcé par un tissu fixé ou collé à la surface intérieure ou extérieure du diaphragme.

11. Seringue selon l'une quelconque des revendications 1 à 10, comprenant en outre une butée (47) disposée à ou par-dessus l'extrémité aval du conduit de façon que ladite butée empêche une course excessive du diaphragme (31) lorsque celui-ci vient dans sa position dynamique, convexe vers l'extérieur.

12. Seringue selon l'une quelconque des revendications 1 à 11, dans laquelle le conduit est constitué par un tube à chocs allongé cylindrique (25).

13. Seringue selon la revendication 12, dans laquelle le tube à chocs (25) comporte un orifice (38) pour évacuer des gaz depuis celui-ci.

14. Seringue selon la revendication 12, comprenant en outre une chambre de rupture (24) ayant une première ouverture destinée à être mise en interface avec un moyen d'excitation (23), et une seconde ouverture qui est en communication de fluide avec l'extrémité amont du conduit.

15. Seringue selon la revendication 14, dans laquelle la seconde ouverture de la chambre de rupture est fermée par une membrane déchirable (26).

16. Seringue selon la revendication 12, comprenant en outre :
(a) une chambre de rupture (24) ayant une ouverture amont et une ouverture aval, l'ouverture amont de ladite chambre de rupture étant apte à être mise en interface avec un moyen d'excitation (23) ; et
(b) une portion cylindrique convergente (5) intercalée entre la chambre de rupture (24) et le conduit (25), ladite portion cylindrique convergente (5) ayant une ouverture amont en communication de fluide avec l'ouverture aval de la chambre de rupture (24), et une ouverture aval en communication de fluide avec l'extrémité amont du conduit, et en outre l'ouverture amont de ladite portion cylindrique convergente (5) étant fermée par une membrane déchirable (26).

17. Seringue selon la revendication 16, dans laquelle la chambre de rupture (24) a un diamètre qui est au moins environ 1,5 fois plus grand que celui du tube à chocs (25).

18. Seringue selon l'une quelconque des revendications 1 à 17, dans laquelle la surface extérieure du diaphragme (31) est collante.

19. Seringue sans aiguille permettant une accélération de particules vers une surface tissulaire visée d'un sujet vertébré, ladite seringue comprenant :
(a) un corps (25) comportant un conduit, ledit conduit ayant une extrémité amont et une extrémité aval, et l'extrémité amont du conduit pouvant être mise en interface avec un moyen d'excitation (23) ; et
(b) une pluralité de diaphragmes (31), ayant une configuration globale présentant l'aspect d'un chapeau haut de forme, disposés au voisinage immédiat de l'extrémité aval du conduit, chaque dit diaphragme (31) ayant une surface intérieure orientée vers le conduit et une surface extérieure, lesdits diaphragmes (31) étant mobiles entre une position initiale dans laquelle un espace concave est créé sur la surface extérieure desdits diaphragmes et une position dynamique dans laquelle la surface extérieure desdits diaphragmes est sensiblement convexe.

20. Seringue selon la revendication 19, dans laquelle la pluralité de diaphragmes (31) sont disposés en série.

21. Seringue selon la revendication 20, dans laquelle la pluralité de diaphragmes (31) sont disposés en série linéaire.

22. Seringue selon l'une quelconque des revendications 19 à 21, dans laquelle l'extrémité aval du conduit s'élargit jusqu'à former une chambre en arrière des surfaces amont de ladite pluralité de diaphragmes (31).

23. Seringue selon l'une quelconque des revendications 19 à 22, dans laquelle une région centrale de la surface extérieure d'au moins un desdits diaphragmes (31) est sensiblement plate ou plane.

24. Seringue selon l'une quelconque des revendications 19 à 23, dans laquelle la surface extérieure d'au moins un desdits diaphragmes (31) comporte un ou plusieurs reliefs (39) qui retiennent de manière sélective des particules sur la surface extérieure dudit diaphragme (31) lorsqu'il est dans sa position initiale.

25. Seringue selon la revendication 24, dans laquelle le ou les reliefs sur la surface extérieure du diaphragme sont constitués par une poche (40) servant à retenir des particules.

26. Seringue selon la revendication 25, dans laquelle ledit/lesdits reliefs sur la surface extérieure du diaphragme sont constitués par une structure (44) suspendue à et d'étendant vers l'extérieur depuis la surface extérieure dudit diaphragme.

27. Seringue selon l'une quelconque des revendications 19 à 26, dans laquelle le conduit est constitué par un tube à onde de choc allongé cylindrique (25).

28. Seringue selon la revendication 27, dans laquelle le tube à chocs (25) comporte une ouverture (38) pour évacuer les gaz depuis celui-ci.

29. Seringue selon la revendication 27, comprenant en outre une chambre de rupture (24) ayant une première ouverture apte à être mise en interface avec un moyen d'excitation (23), et une seconde ouverture qui est en communication de fluide avec l'extrémité amont du conduit.

30. Seringue selon la revendication (29), dans laquelle la seconde ouverture de la chambre de rupture (24) est fermée par une membrane déchirable (26).

31. Seringue selon la revendication 27, comprenant en outre :
(a) une chambre de rupture (24) ayant une ouverture amont et une ouverture aval, l'ouverture amont de ladite chambre de rupture (24) étant destinée à être mise en interface avec un moyen d'excitation (23) ; et
(b) une portion cylindrique convergente (5) intercalée entre la chambre de rupture (24) et le conduit, ladite portion cylindrique convergente (5) ayant une ouverture amont en communication de fluide avec l'ouverture aval de la chambre de rupture (24), et une ouverture aval en communication de fluide avec l'extrémité amont du conduit, et l'ouverture aval de ladite portion cylindrique convergente (5) étant en outre fermée par une membrane déchirable (26).

32. Seringue selon l'une quelconque des revendications 29 à 31, dans laquelle la chambre de rupture a un diamètre qui est au moins environ 1,5 fois plus grand que celui du tube à onde de choc (25).

33. Seringue sans aiguille permettant une accélération de particules jusqu'à une surface tissulaire visée d'un sujet vertébré, ladite seringue comprenant :
(a) un corps (25) comportant un conduit, ledit conduit ayant une extrémité amont et une extrémité aval ;
(b) une chambre de rupture (24) ayant une ouverture amont et une ouverture aval, l'ouverture amont de ladite chambre de rupture étant apte à être mise en interface avec un moyen d'excitation (23) ;
(c) une portion cylindrique convergente (5) intercalée entre la chambre de rupture (24) et le conduit, ladite portion cylindrique convergente (5) ayant une ouverture amont en communication de fluide avec l'ouverture aval de la chambre de rupture (24), et une ouverture aval en communication de fluide avec l'extrémité amont du conduit, et en outre l'ouverture amont de ladite portion cylindrique convergente (5) étant fermée par une membrane déchirable (26) ; et
(d) un diaphragme (31) disposé au voisinage immédiat de l'extrémité aval du conduit, ledit diaphragme (31) ayant une surface interne en regard de l'ouverture et une surface externe, ledit diaphragme (31) étant mobile entre une position initiale dans laquelle un espace concave est créé sur la surface extérieure du diaphragme (31) et une position dynamique dans laquelle la surface extérieure du diaphragme (31) est sensiblement convexe, ledit diaphragme (31) ayant une configuration générale ayant la configuration d'un chapeau haut de forme.

34. Seringue selon la revendication 33, dans laquelle le conduit est constitué par un tube à onde de choc allongé cylindrique (25).

35. Seringue selon la revendication 34, dans laquelle la chambre de rupture (24) a un diamètre au moins environ 1,5 fois plus grand que celui du tube à onde de choc (25).
